# EUROPEAN PATENT APPLICATION

(11) **EP 1 932 583 A1**
(43) Date of publication of application: **18.06.2008**
(21) Application number: 06124945.4
(22) Date of filing: 28.11.2006
(51) Int. Cl.: B01J 4/00, B01J 19/26

(54) **Reactor vessel**

(71) Applicant: SHELL INTERNATIONALE RESEARCH MAATSCHAPPIJ B.V., 2596 HR Den Haag (NL)
(72) Inventor: Dann, Yvette Louise, 4780 LN Moerdijk (NL); Klusener, Peter Anton August, 1031 CM Amsterdam (NL); Lee, Ming Chiann, 1031 CM Amsterdam (NL)
(74) Representative: Zeestraten, Albertus W. J.

(57) **Abstract**

The invention relates to a reactor vessel having a lower part and two opposite ends, which reactor vessel comprises a liquid inlet at one end, a fluid outlet at the opposite end and a gas inlet device arranged in the lower part, wherein the gas inlet device includes one or more perforated pipes of which the hole pitch is more than 15 times the hole diameter. Further, the present invention relates to a reactor system including such reactor vessel and to a process such reactor vessel is used for.

## Description

The present invention relates to a reactor vessel, especially a reactor vessel for contacting a liquid reactant, such as ethylbenzene or cumene (isopropylbenzene), with a gaseous reactant, such as oxygen-containing gas in order to obtain an organic hydroperoxide. Further, the present invention relates to a reactor system including such reactor vessel and to a process such reactor vessel is used for.

Reactor vessels for contacting a liquid reactant with a gaseous reactant are known in the art and have been described for example in US-A-4,269,805.

There is still room for improving reactor vessels for contacting gaseous and liquid reactant. A better contact between the gaseous and the liquid reactants generally is desirable, as this tends to make the reaction of the liquid reactant and the gaseous reactant more efficient. A higher efficiency can make it possible to operate the process at higher throughput. A further advantage of better contact between the gaseous and the liquid reactant can be reduction of the amount of by-products formed. By-product formation can be caused by heating liquid reactant in the absence of sufficient gaseous reactant. Less by-product formation will generally give an increase in the amount of desired product.

A process in which liquid reactant is contacted with gaseous reactant is the reaction of liquid organic compounds with oxygen-containing gas in order to obtain the corresponding organic hydroperoxide. Although the organic compound in such oxidation reaction can in principle be any compound, organic compounds most frequently used are alkylarene compounds which upon oxidation, are converted into the corresponding alkylarene hydroperoxides. Alkylarene compounds most frequently used are benzene compounds containing at least 1 alkyl substituent which alkyl substituent contains of from 1 to 10 carbon atoms, preferably of from 2 to 8 carbon atoms. Preferably, the benzene compound contains on average of from 1 to 2 constituents. The alkylarene compounds most frequently encountered are ethylbenzene, cumene and di(isopropyl)benzene.

Ethylbenzene hydroperoxide is commercially prepared by the liquid phase oxidation of ethylbenzene with oxygen-containing gas, such as air. Such oxidation processes are well known in the art. An example thereof is described in US-A-5,883,268.

In such oxidation reaction of ethylbenzene, methyl phenyl carbinol (1-phenylethanol) and methyl phenyl ketone (acetophenone) are formed as side-products. Subsequent oxidation of an alkene (such as propene) with the ethylbenzene hydroperoxide results in the production of alkene oxide (an oxirane or epoxide; such as propylene oxide) and methyl phenyl carbinol. Methyl phenyl ketone can be converted with hydrogen into methyl phenyl carbinol. Methyl phenyl carbinol can be dehydrated into styrene. Both styrene and propylene oxide are valuable market products.

Processes for the joint preparation of styrene monomer ("SM") and propylene oxide ("PO") are known in the art and are commonly referred to as "SM/PO" processes. An SM/PO process is for example described in WO 00/05186. In general, an SM/PO process comprises the steps of:
(a) reacting ethene and benzene to form ethylbenzene,
(b) reacting ethylbenzene with oxygen-containing gas to form ethylbenzene hydroperoxide,
(c) reacting ethylbenzene hydroperoxide with propene in the presence of an epoxidation catalyst to form propylene oxide and 1-phenylethanol, and
(d) dehydrating 1-phenylethanol into styrene in the presence of a suitable dehydration catalyst.

Cumene hydroperoxide is applied commercially for preparing phenol and acetone. Alternatively, cumene hydroperoxide can be reacted with propene to obtain propylene oxide in a process similar to the above SM/PO process in which ethylbenzene is used. The main difference between the cumene based process and the ethylbenzene based SM/PO process resides in the fact that the alcohol derived from cumene hydroperoxide which is formed upon reaction of cumene hydroperoxide and propene, generally is hydrogenated back to cumene.

The oxygen-containing gas may be introduced into the oxidation reactor or reactor vessel in any way, for example by means of sparge pipes. Sparge pipes are normally mounted at the bottom of the reactor vessel. They comprise openings or holes in their walls through which a gas or gas mixture may be fed into the reaction mixture. Sparge pipes can be characterised by the size (diameter) of their holes and by the hole pitch. The hole pitch is the distance between the centres of two adjacent holes. A further characteristic of sparge pipes is the ratio of hole pitch to hole diameter.

WO 2005/085191 discloses the use of sparge pipes for providing oxygen-containing gas in a process for the production of cumene hydroperoxide from cumene wherein the oxygen concentration in the total amount of the gas fed into the liquid phase of the reactor is adjusted to the range of 22 mol% to 50 mol%, which range lies above the oxygen concentration in air. According to WO 2005/085191, sparge pipes (or spargers) are used of which the ratio of hole pitch to hole diameter is at least two, more preferably at least four. The situation where the hole pitch to hole diameter is two, is a situation where the minimum distance between the peripheries of the normally circular holes is equal to the hole diameter.

It is stated in said WO 2005/085191 that by using such sparge pipes, the reaction system is prevented from forming an explosive composition when feeding an oxygen-containing gas high in oxygen concentration into the reactor, thereby enabling to improve safety. In the Examples of WO 2005/085191, a ring-like sparge pipe comprising 25 holes is used which has a hole diameter of 2 mm, a hole pitch of 10 mm and therefore a ratio of hole pitch to hole diameter of 5. WO 2005/085191 further requires that the hole diameter is relatively small so as to further reduce the explosion risk. A maximum hole diameter of 8.0 mm is mentioned. Further, WO 2005/085191 mentions an upper limit for the hole pitch which is 15 times the hole diameter.

It has now surprisingly been found that the performance of a reactor vessel for contacting liquid reactant with gaseous reactant can be improved greatly in an easy and simple way.

The present invention relates to a reactor vessel having a lower part and two opposite ends, which reactor vessel comprises a liquid inlet at one end, a fluid outlet at the opposite end and a gas inlet device arranged in the lower part, wherein the gas inlet device includes one or more perforated pipes of which the hole pitch is more than 15 times the hole diameter.

Sparge pipes or perforated pipes comprise openings or holes in their walls through which a gas or gas mixture may be fed into the reaction mixture. The hole pitch is the distance between the centres of two adjacent holes of such sparge or perforated pipes. Further, where the perforated pipe or pipes used in the present invention, comprise non-circular holes, the hole diameter is the length of the longest straight line connecting two points on the hole periphery.

Preferably, the perforated pipe or pipes used in the present invention are mounted at or near the bottom of the reactor vessel.

According to the present invention, the hole diameter of the sparge or perforated pipes may be from 1 to 8 mm, more suitably from 1 to 6 mm, more suitably from 2 to 4 mm and even more suitably around 3 mm. Further, the hole pitch may be from 25 to 300 mm and suitably from 50 to 250 mm; and the ratio of hole pitch to hole diameter may be from more than 15 to 200, suitably from 25 to 150 and more suitably from 35 to 150. However, hole diameters, hole pitches and ratios of hole pitch to hole diameter falling outside the above ranges can also be applied in the present process, provided that the hole pitch is more than 15 times the hole diameter.

In addition to preventing the formation of an explosive composition as mentioned above, it has been found by the present inventors that additional advantages are associated with using a relatively large hole pitch, especially when using a hole pitch which is considerably larger than the hole pitch disclosed in the Examples of WO 2005/085191. The main additional advantage is that the reaction rate is improved by using a relatively large hole pitch. Without wishing to be bound by theory, it is believed that the higher reaction rate is caused by preventing coalescence of two or more bubbles containing oxygen-containing gas into bigger bubbles. Since the bigger bubbles have relatively less surface area for the oxygen to contact and react with the oxidisable organic compound, it is believed that such bigger bubbles retard the oxidation reaction. It is believed that such coalescence can be prevented as much as possible if the distance between two adjacent holes is relatively large thereby decreasing the chance of contact and coalescence between the bubbles.

Another advantage of preventing the formation of relative large gas bubbles is that more efficient use is made of the oxygen fed into the reaction mixture, which corresponds to an increase in the ratio of the total amount of organic hydroperoxide produced to the total amount of oxygen gas fed. Further, by increasing the percentage of oxygen gas taking part in the oxidation reaction, a build-up of oxygen gas in the gas outlet stream, which leads to a higher explosion risk, is prevented as well.

In view of the foregoing, it is advantageous that in the perforated pipe or pipes present in the reactor vessel according to the present invention, the hole pitch is more than 15 times the hole diameter, more preferably at least 20 times the hole diameter, more preferably at least 25 times the hole diameter, more preferably at least 30 times the hole diameter, more preferably at least 35 times the hole diameter, more preferably at least 40 times the hole diameter, and most preferably at least 50 times the hole diameter. Preferably, the hole pitch is at most 200 times, more preferably at most 175 times the hole diameter, more preferably at most 150 times the hole diameter, more preferably at most 125 times the hole diameter, and most preferably at most 100 times the hole diameter.

The reactor vessel of the present invention may be a substantially horizontal reactor. By substantially horizontal is understood substantially parallel to the plane of the horizon. Preferably, the reactor vessel for use in the present invention is tubular. Such a tubular reactor vessel can have a wide variety of shapes. For example, such a tubular reactor vessel can have a square, rectangular, circular or elliptical cross-section. For practical purposes a reactor vessel with a circular cross-section is preferred.

In a horizontal reactor the majority of the fluid flows in horizontal direction during normal operation. Horizontal reactor vessels make it possible to apply long residence times and to contact the liquid with a relatively large amount of gas. This is advantageous in the manufacture of organic hydroperoxide in view of the relatively low reaction rate.

The gas inlet device, including one or more perforated pipes, is arranged in the lower part of the reactor vessel. By the lower part of a horizontal reactor vessel is understood that part of the reactor vessel lying below the horizontal plane through the central longitudinal axis of the horizontal reactor vessel.

As described herein further, a single reactor vessel can comprise several reaction zones. If this is the case, it is preferred that each reaction zone contains a gas inlet device including one or more perforated pipes. Preferably, each gas inlet device can be operated independently in such case.

The reactor vessel according to the present invention is especially suitable for contacting a liquid reactant and a gaseous reactant. Therefore, the present invention further relates to a process of contacting a liquid reactant with a gaseous reactant which process is carried out in a reactor vessel having a lower part and two opposite ends, which process comprises adding the liquid reactant to the reactor vessel via a liquid inlet at one end of the reactor vessel, adding the gaseous reactant via a gas inlet device arranged in the lower part and removing reaction product via a fluid outlet at the opposite end, which process is carried out in a reactor vessel wherein the gas inlet device includes one or more perforated pipes of which the hole pitch is more than 15 times the hole diameter. Preferably, such process is a process of manufacture of an organic hydroperoxide wherein a liquid organic compound is contacted with an oxygen-containing gas. Preferably, said organic compound is cumene and/or ethylbenzene. Most preferably, ethylbenzene is used.

Such oxidation of an organic compound into organic hydroperoxide can be carried out by any suitable process known in the art wherein the oxidisable compound (for example ethylbenzene) is in the liquid phase and the oxidant is an oxygen-containing gas. In general, the liquid phase oxidation of ethylbenzene into ethylbenzene hydroperoxide is carried out at a temperature of from 50 to 250 °C, suitably of from 100 to 200 °C, and more suitably of from 120 to 180 °C. The reactor vessel will generally contain a heat exchanger so as to heat the reaction mixture at the start of operation and to cool when the reaction has progressed sufficiently.

In the above-mentioned oxidation, the amount of oxygen to be added and the amount of organic compound (for example ethylbenzene) to be added depend on the specific circumstances of the process such as the volume and shape of the reactor vessel and the concentration of hydroperoxide which should preferably be kept below 20 wt.% on the basis of the total weight of the reaction mixture. Suitably, the ethylbenzene hydroperoxide concentration in the reaction mixture is lower than 15 wt.%, more suitably lower than 12 wt.%. The ethylbenzene hydroperoxide concentration in the reaction mixture may be in the range of from 10 to 15 wt.%, more suitably 10 to 12 wt.%.

The pressure of the above-mentioned oxidation process is not critical and can be chosen such as to best accommodate specific circumstances. Generally, the pressure near the top of the reactor vessel will be from atmospheric to 10 * 10⁵ N/m², more specifically from 1 to 5 * 10⁵ N/m².

The oxidation of organic compound (for example ethylbenzene) is carried out in the liquid phase and can be carried out in the presence of a diluent. This diluent is preferably a compound which is liquid under the reaction conditions and does not react with the starting materials and product obtained. The diluent can be the organic compound to be oxidised. Preferably, a solution of ethylbenzene hydroperoxide in ethylbenzene is prepared.

The process according to the present invention may be carried out continuously. The present reactor vessel used in a continuous process for oxidation of organic compounds such as ethylbenzene, is provided with a liquid inlet at one end and a fluid outlet at the opposite end. Further, the lower part of the reactor vessel contains a gas inlet device, which includes one or more sparge pipes (or perforated pipes). Normally, the gas is removed together with the liquid, via the fluid outlet. However, dependent on the exact circumstances, it can be advantageous to remove excess gas via a separate gas outlet during normal operation. One or more separate gas outlets can be present. Normally, the fluid outlet is at the bottom of the vessel and the optional gas outlet at the top of the vessel. However, this is not required. The preferred height at which each outlet is situated depends on a number of factors as will be appreciated by someone skilled in the art. One of these circumstances is the level, which the liquid generally reaches.

The above-mentioned oxidation is carried out by feeding the oxygen-containing gas to the reaction mixture as a gas inlet stream. The oxygen concentration in the gas feed may be from 5 to 100 vol.%, suitably from 10 to 60 vol.%, more suitably from 20 to 50 vol.%, wherein the remainder is preferably an inert gas such as nitrogen. Air, which on average contains 21 vol.% of oxygen is the preferred oxygen-containing gas feed. The temperature of the gas at the gas inlet may be from ambient temperature to 250 °C.

The temperature of the gas in the gas outlet stream is normally higher than ambient temperature and may be as high as the reactor temperature. To avoid the risk of explosion where an oxygen-containing gas is used, the oxygen concentration in the gas outlet stream, after cooling to ambient temperature, is normally lower than 10 vol.%, suitably lower than 8 vol.%, and more suitably lower than 7 vol.%. For example, the gas outlet stream may comprise 5 vol.% of oxygen.

The present process may be carried out continuously. In a continuous oxidation process, the oxidation reactor is preferably horizontally oriented which means that the stream of reaction mixture flows horizontally through the reactor. Preferably, where the oxidation reactor is horizontally oriented, a substantially horizontal reactor vessel is used having a lower part and two opposite ends, which reactor vessel comprises a liquid inlet at one end, a fluid outlet at the opposite end and a gas inlet device arranged in the lower part, which reactor vessel contains at least one substantially vertical baffle-plate arranged in the direction of liquid flow through the reactor vessel during normal operation. Such a reactor vessel is disclosed in WO 2006/024655. The reactor vessel disclosed therein may be used in the present invention provided that one or more perforated pipes are used as part of the gas inlet device, wherein the hole pitch of said pipe or pipes is adjusted such that the hole pitch is more than 15 times the hole diameter.

Further, in such continuous process, the oxidation reactor may be comprised of two or more separate reaction zones (sometimes also referred to as separate compartments). Alternatively, two or more oxidation reactors arranged in series may be used of which some or all may be comprised of two or more separate reaction zones. In such case, the fluid outlet of one reactor vessel is connected to the liquid inlet of a subsequent reactor vessel. Therefore, the present invention also relates to a reactor system that includes at least 2 reactor vessels arranged in series in which at least one of the reactor vessels is according to the present invention.

Where the oxidation reactor is comprised of two or more separate reaction zones, the stream of reaction mixture flows from the first reaction zone to the second reaction zone and from said second reaction zone to a further reaction zone or to a further reactor or to a separation unit. The reaction zones can differ from each other in various aspects such as the degree of conversion, which has taken place. The separate reaction zones can be created in a single reactor vessel by means, which are known to someone skilled in the art. A very well known means to achieve this involves placing a vertical plate between the reaction zones perpendicular to the direction of flow such that the plate has an opening, which permits fluid to flow from one reaction zone to the subsequent reaction zone. A detailed set-up of a single reactor vessel containing a plurality of reaction zones has been described in US-A-4,269,805. Such a reactor vessel can be used in the present invention provided that one or more perforated pipes are used as part of a gas inlet device, wherein the hole pitch of said pipe or pipes is adjusted such that the hole pitch is more than 15 times the hole diameter.

Each of said reaction zones and said reactors may be provided with sparge pipes of the above-mentioned type, which may be mounted at or near the bottom of the reactor(s). Depending on the kinetics of the reaction system, more oxygen may be needed in the last reaction zone or reactor. This is for example the case in the oxidation of ethylbenzene, which is an autocatalytic reaction. Since in such case in the last reaction zone or reactor (for example for the oxidation of ethylbenzene), the conversion is higher than in the first reaction zone or reactor, more oxygen is needed in the last reaction zone or reactor. This may be achieved by varying the hole diameter in the sparge pipes from reaction zone to reaction zone and/or from reactor to reactor, and/or by varying the number of holes in the sparge pipes from reaction zone to reaction zone and/or from reactor to reactor through adjusting the hole pitch. In order to introduce more oxygen into the last reaction zone or reactor, the number of sparge holes may be adjusted such that it is larger in the last reactor or reaction zone than in the first reactor or reaction zone. The number of holes can be increased by lowering the hole pitch (when keeping the hole diameter constant). However, preferably, care should be taken that in such last reactor or reaction zone the hole pitch is still more than 15 times the hole diameter, for the reasons as elucidated above. Alternatively, the number of holes can be increased by using additional sparge pipes.

In general, in cases where a reactor vessel is used for contacting a liquid reactant with a gaseous reactant added via a gas inlet device arranged in the lower part of the reactor vessel wherein the gas inlet device includes one or more perforated pipes, of which the hole pitch may be less than, equal to or more than 15 times the hole diameter, it is aimed at a homogeneous distribution of the gaseous reactant through the reactor vessel. In practice, this is however difficult to achieve, as is further illustrated below with reference to the oxidation of ethylbenzene with air into ethylbenzene hydroperoxide.

Where a reactor vessel is used for the oxidation of ethylbenzene with air into ethylbenzene hydroperoxide, there may be zones within the reactor vessel at specific locations where substantially no oxygen is present. These zones are also sometimes referred to as "oxygen-depleted" or "oxygen-starvation" zones. In the substantial absence of oxygen within such zones, the oxidation of ethylbenzene into ethylbenzene hydroperoxide hardly takes place and side reactions, such as the conversion of ethylbenzene hydroperoxide into methyl phenyl carbinol (1-phenylethanol) and methyl phenyl ketone (acetophenone), prevail. Therefore, where the portion of the total reaction mixture volume occupied by the oxygen-starvation zone or zones is enlarged, the amount of methyl phenyl carbinol and methyl phenyl ketone in the fluid leaving the reactor will be increased, whereas the amount of ethylbenzene hydroperoxide will be decreased.

In processes for the joint preparation of styrene monomer ("SM") and propylene oxide ("PO"), commonly referred to as "SM/PO" processes, methyl phenyl ketone can be converted with hydrogen into methyl phenyl carbinol, and methyl phenyl carbinol can be dehydrated into styrene. Methyl phenyl ketone and methyl phenyl carbinol cannot be used as an oxidant for oxidising propene into propylene oxide. On the other hand, ethylbenzene hydroperoxide is used to oxidise propene yielding both propylene oxide and methyl phenyl carbinol which, in such SM/PO process, is eventually converted into styrene. In other words, ethylbenzene hydroperoxide leaving the oxidation reactor will eventually yield both styrene and propylene oxide, whereas methyl phenyl ketone and methyl phenyl carbinol leaving the oxidation reactor will eventually only yield styrene.

Both styrene and propylene oxide are valuable market products. The demands for these two products in the market however fluctuate differently in time. It can be deduced from the above that the ratio of styrene to propylene oxide formed within an integrated process, such as the SM/PO process, may be varied by varying the ratio of the amount of ethylbenzene hydroperoxide in the fluid leaving the reactor(s) used for the production of ethylbenzene hydroperoxide to the amount of methyl phenyl carbinol and methyl phenyl ketone in said same fluid. The latter ratio may be varied by varying the portion of the total reaction mixture volume occupied by the oxygen-starvation zone or zones. Enlarging said portion will result in an increase of the amount of methyl phenyl carbinol and methyl phenyl ketone in the fluid leaving said reactor(s), and a decrease of the amount of ethylbenzene hydroperoxide in said same fluid.

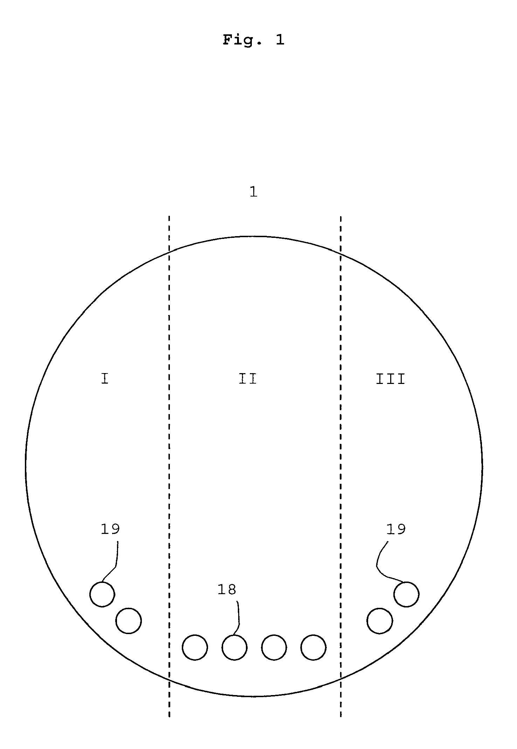

The above will be further illustrated with reference to Figure 1 showing a cross-section through a horizontally oriented, cylindrical (or tubular) reactor used for the production of ethylbenzene hydroperoxide from ethylbenzene and air, which cross-section is perpendicular to the direction of flow of the reaction mixture through the reactor. This reactor is part of an integrated SM/PO process as referred to above. In a horizontal or horizontally oriented reactor, the stream of reaction mixture flows horizontally through the reactor. An example of such reactor is described in above-mentioned WO 2006/024655.

The lower part of the reactor vessel 1 shown in Figure 1 contains a gas inlet device which includes a total number of eight perforated pipes 18 and 19 through which perforations or holes, air is introduced into the reactor vessel. The four perforated pipes 18 are mounted at or near the bottom of the reactor within middle part II of the reactor as designated in Figure 1. Further, two of the four perforated pipes 19 are mounted at or near the bottom of the reactor within side part I of the reactor as designated in Figure 1, whereas the remaining two are mounted at or near the bottom of the reactor within side part III of the reactor as designated in Figure 1. The imaginary boundaries between side part I and middle part II and between middle part II and side part III are indicated by the vertical dashed lines in Figure 1. However, in practice, the middle part may be smaller and the side parts consequently bigger than middle part II and side parts I and III, respectively, as depicted in Figure 1, or vice versa. Further, in each of parts I to III, the number of perforated pipes may be varied. Preferably, the number of perforated pipes 19 in side part I is the same as the number of perforated pipes 19 in side part III.

The length of each of the perforated pipes 18 and 19 shown in Figure 1 is substantially the same. Therefore, in case where the air pressure in each of those pipes is the same, as well as the number of holes, the hole pitch and the hole diameter, the amount of air introduced into the reactor per hour via the four perforated pipes 18 within middle part II is the same as the amount of air introduced into the reactor per hour via the four perforated pipes 19 within side part I and side part III together. In such case, it may appear that the portion of the total reaction mixture volume occupied by the oxygen-starvation zone or zones, is either too large or too small. Referring to Fig. 1, this may be changed by varying the ratio of the amount of air introduced into the reactor per hour via the perforated pipes 18 within middle part II to the amount of air introduced into the reactor per hour via the perforated pipes 19 within side part I and side part III together. This ratio may for example be increased by increasing the air pressure within the perforated pipes 18 only and/or by increasing the hole diameter in the perforated pipes 18 only and/or by increasing the number of holes, for example by decreasing the hole pitch, in the perforated pipes 18 only or by including more perforated pipes within middle part II than within side part I and side part III together. In some instances, it may be preferred either to increase or to decrease said ratio, depending on whether it is desired that, in the integrated SM/PO process as referred to above, the total production of styrene is increased or the total production of propylene oxide is increased. Where it is desired to increase said ratio for a reactor similar to the one shown in Figure 1, said ratio may be increased from 1:1 (normal operation) up to 1:7 and even higher and an optimum may for example be found between 1:1.25 and 1:4.

## Claims

1. Reactor vessel having a lower part and two opposite ends, which reactor vessel comprises a liquid inlet at one end, a fluid outlet at the opposite end and a gas inlet device arranged in the lower part, wherein the gas inlet device includes one or more perforated pipes of which the hole pitch is more than 15 times the hole diameter.

2. Reactor vessel according to claim 1, wherein the hole pitch is from 25 to 150 times the hole diameter.

3. Reactor vessel according to claim 2, wherein the hole pitch is from 35 to 150 times the hole diameter.

4. A reactor system that includes at least two reactor vessels arranged in series in which at least one of the reactor vessels is according to any one of the preceding claims.

5. A process of contacting a liquid reactant with a gaseous reactant which process is carried out in a reactor vessel having a lower part and two opposite ends, which process comprises adding the liquid reactant to the reactor vessel via a liquid inlet at one end of the reactor vessel, adding the gaseous reactant via a gas inlet device arranged in the lower part and removing reaction product via a fluid outlet at the opposite end, which process is carried out in a reactor vessel wherein the gas inlet device includes one or more perforated pipes of which the hole pitch is more than 15 times the hole diameter.

6. Process of manufacture of an organic hydroperoxide which process comprises contacting a liquid organic compound with an oxygen-containing gas in a process according to claim 5.

7. Process according to claim 6, in which process the organic compound is ethylbenzene.
